# EUROPEAN PATENT APPLICATION

(11) **EP 4 549 549 A1**
(43) Date of publication of application: **07.05.2025**
(21) Application number: 23206757.9
(22) Date of filing: 30.10.2023
(51) Int. Cl.: C12N 5/071

(54) **ENDOTHELIAL CELL DIFFERENTIATION**

(71) Applicant: Stimuliver ApS, 2200 København N (DK)
(72) Inventor: SZKOLNICKA, Dagmara, 2200 København N (DK); GONZÁLEZ DEL BARRIO, Lydia, 2200 København N (DK); QUINTANA CALDERÓN, Carlos, 2200 København N (DK); C. HAY, David, 2200 København N (DK)
(74) Representative: Zacco Denmark A/S

(57) **Abstract**

The present invention relates to methods for producing endothelial cells and the cells obtained from the method. The invention further relates to methods of producing liver an aggregation of liver cells comprising the endothelial cells of the present invention.

## Description

### Field of the invention

The present invention relates to methods for producing endothelial cells and the cells obtained from the method. The invention further relates to methods of producing liver, an aggregation of liver cells comprising the endothelial cells of the present invention.

### Background of the invention

Stem cell-based tissue engineering applications aim to capture key features of development, for example to promote *ex vivo* tissue formation for the replacement of malfunctioning/defective or injured tissues. Stem cells, in particular pluripotent stem cells, are useful for such applications due to their capacity to self-renew and differentiate into a plurality of differentiated cells types.

Endothelial cells form the barrier between vessels and tissues and control the flow of substances and fluid into and out of a tissue. Thus, endothelial cells are of importance for the exchange of substances including oxygen, water, and lipids, as well as in the transfer of carbon dioxide and urea from a tissue to a vessel. Due to the capacity to differentiate into all capillary niches, endothelial progenitor cell is of great interest in vascularizing engineered tissues, such as liver tissue engineering for use in regenerative medicine. There is therefore a need in the art for improved methods for producing endothelial progenitor cells.

### Summary of the invention

The object of the present invention is to provide an improved method for producing endothelial cells. A further object of the present invention is to provide an improved method for producing an aggregation of liver cells.

In a first aspect, the present invention provides a method for producing endothelial cells, comprising:
seeding pluripotent stem cells on a cell culture substrate comprising:
   (i) laminin-521 or a fragment of laminin-521;
   (ii) laminin-111 or a fragment of laminin-111; and
culturing the pluripotent stem cells in the presence of one or more differentiation factors that stimulates endothelial cell differentiation of said pluripotent stem cells to obtain said endothelial cells.

In further aspects, the present invention provides endothelial cells obtained by the method according to the invention and a composition comprising said endothelial cells.

In yet a further aspect, the present invention provides method for producing an aggregation of liver cells, said method comprising the step of:
(i) providing endothelial cells obtained by the method of the present invention for producing endothelial cells;
(i) providing hepatic progenitor cells;
(ii) prepare a mixture comprising said hepatic progenitor cells and endothelial cells in a culture medium;
(iii) seed the mixture on a cell culture substrate, such as in substrate in a microwell format;
(iv) allow the mixture of cells to propagate and form an aggregation of cells;
(v) collect said aggregation of cells.

The invention further provides an aggregation of liver cells obtained by the method of the present invention and the aggregation of liver cells for use as a medicament.

### Brief description of the drawings

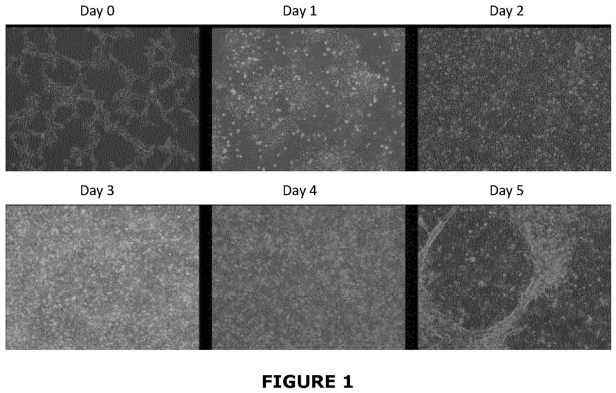

**Figure 1****.** Phase contrast microscopy images displaying cell morphology throughout the differentiation process of stem cells to endothelial cells. Magnification x10.

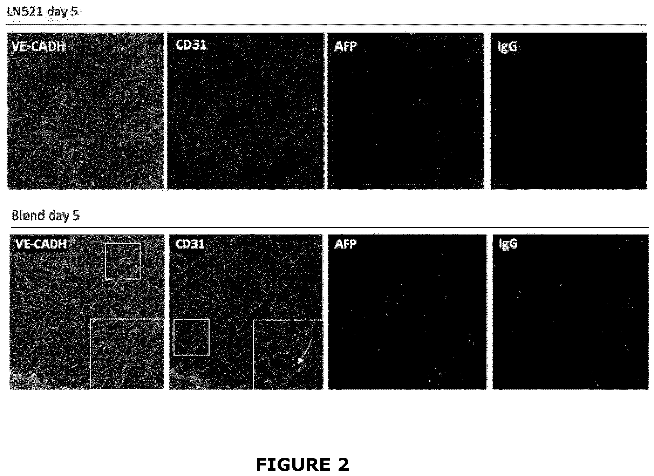

**Figure 2****.** Endothelial cells - markers at day 5. Immunofluorescent images of endothelial cells on day 5 of differentiation. Cells are positive for VE-cadherin (VE-CADH) and cluster of differentiation 31 (CD31) and negative for Alpha-fetoprotein (AFP) markers. IgG was used as a negative control. White squares indicate zoom of a specific area. Arrow points at CD31 expression. The data demonstrate that the endothelial cells obtained by seeding the pluripotent stem cells on a blend of laminin 521 and laminin 111 display a more defined/intense VE-cadherin (VE-CADH) and cluster of differentiation 31 (CD31) immunostaining.

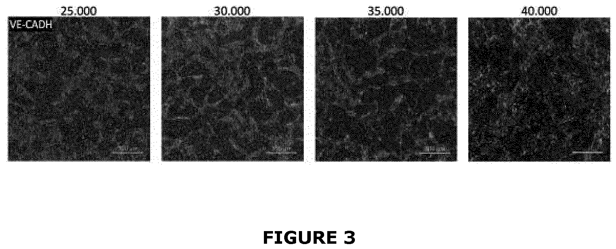

**Figure 3****.** Immunofluorescent images of CD144⁺ endothelial cells (VE-CADH) throughout different initial hESC seeding densities before differentiation (25,000-40,000 cells/cm²). Optimal hESC seeding density was determined through highest intensity and overall area of CD144⁺ cells (30,000 cells/cm²). Scale 300 µm. Cells seeded at initial 30.000 cells/cm² display the most extensive (in area), uniform and defined (signal is more intense) expression of CD144 (VE-CADH) as opposed to the same cells at an initial seeding density of 25.000, 35.000 and 40.000 cells/cm².

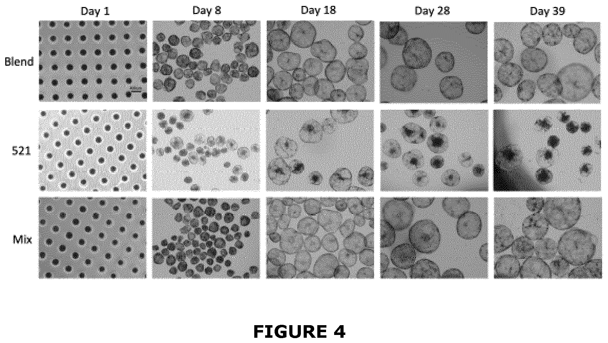

**Figure 4****:** Brightfield microscopy images showing liver cell morphology at 4x magnification. Day 1 demonstrates spheres growing inside of the microwells. The following days demonstrate growth of the free-floating spheres in poly-HEMA coated wells. Days indicate sphere maintenance after cell aggregation. Spheres were generated using either the blend of laminins (Blend), single 521 laminin (521) or a mix of mentioned laminins (Mix). Scale bar indicates 400um. Blend: samples from liver spheres composed of differentiated cells that initially were plated and cultured on laminins 521/111 in a ratio of 1:3. 521: samples from liver spheres composed of differentiated cells that were initially plated and cultured on laminin 521 exclusively. Mix: samples from liver spheres composed of differentiated cells that initially were plated and cultured on laminin 521 (endothelial cells) and on laminins 521/111 (hepatic progenitors) in a ratio 1:3.

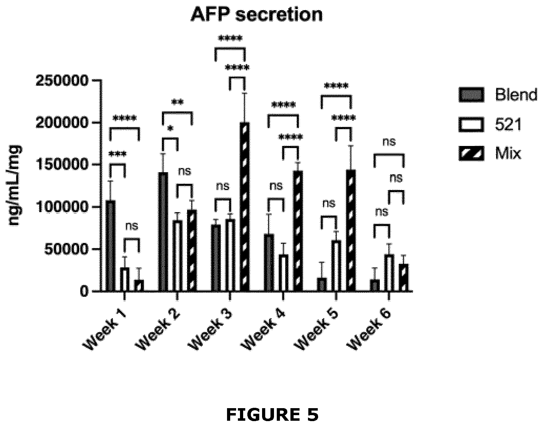

**Figure 5****:** Graph describes secretion of Alpha fetoprotein (AFP) per mg of protein over a period of 6 weeks. Blend data are expressed as mean + SD of 3 independent experiments. 521 data are expressed as mean + SD of 3 biological replicates from one experiment. 2-way ANOVA was performed for each week comparing Blend vs 521; ns P > 0.05; * P < 0.0332; ** P < 0.0021; *** P < 0.0002; **** P < 0.0001. Data were analyzed by Graphpad (Prism 9). Blend = samples from liver spheres composed of differentiated cells that initially were plated and cultured on laminins 521/111 in a ratio of 1:3. 521 = samples from liver spheres composed of differentiated cells that were initially plated and cultured on laminin 521 exclusively. Mix = samples from liver spheres composed of differentiated cells that initially were plated and cultured either on laminin 521 (endothelial cells) or on laminins 521/111 in a ratio 1:3 (hepatic progenitors). Blend: samples from liver spheres composed of differentiated cells that initially were plated and cultured on laminins 521/111 in a ratio of 1:3. 521: samples from liver spheres composed of differentiated cells that were initially plated and cultured on laminin 521 exclusively. Mix: samples from liver spheres composed of differentiated cells that initially were plated and cultured on laminin 521 (endothelial cells) and on laminins 521/111 (hepatic progenitors) in a ratio 1:3.

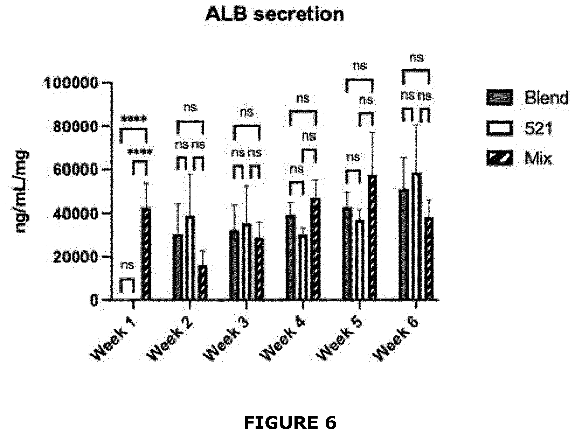

**Figure 6****:** Graph describes secretion of Albumin (ALB) per mg of protein over a period of 6 weeks. Blend data are expressed as mean + SD of 3 independent experiments. 521 data are expressed as mean + SD of 3 biological replicates from one experiment. 2-way ANOVA was performed for each week comparing Blend vs 521; ns P > 0.05; * P < 0.0332; ** P < 0.0021; *** P < 0.0002; **** P < 0.0001. Data were analyzed by Graphpad (Prism 9). Blend = samples from liver spheres composed of differentiated cells that initially were plated and cultured on laminins 521/111 in a ratio of 1:3. 521 = samples from liver spheres composed of differentiated cells that were initially plated and cultured on laminin 521 exclusively. Mix = samples from liver spheres composed of differentiated cells that initially were plated and cultured either on laminin 521 (endothelial cells) or on laminins 521/111 in a ratio 1:3 (hepatic progenitors). Blend: samples from liver spheres composed of differentiated cells that initially were plated and cultured on laminins 521/111 in a ratio of 1:3. 521: samples from liver spheres composed of differentiated cells that were initially plated and cultured on laminin 521 exclusively. Mix: samples from liver spheres composed of differentiated cells that initially were plated and cultured on laminin 521 (endothelial cells) and on laminins 521/111 (hepatic progenitors) in a ratio 1:3.

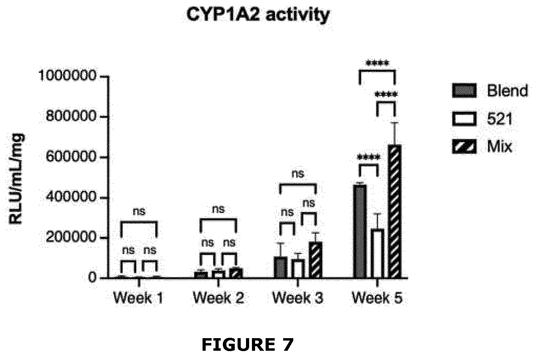

**Figure 7****:** Graph describes activity of Cytochrome P450 1A2 (CYP1A2) per mg of protein over a period of 5 weeks. Blend data are expressed as mean + SD of 3 independent experiments. 521 data are expressed as mean + SD of 3 biological replicates from one experiment. 2-way ANOVA was performed for each week comparing Blend vs 521; ns P > 0.05; * P < 0.0332; ** P < 0.0021; *** P < 0.0002; **** P < 0.0001. Data were analyzed by Graphpad (Prism 9). Blend = samples from liver spheres composed of differentiated cells that initially were plated and cultured on laminins 521/111 in a ratio of 1:3. 521 = samples from liver spheres composed of differentiated cells that were initially plated and cultured on laminin 521 exclusively. Mix = samples from liver spheres composed of differentiated cells that initially were plated and cultured either on laminin 521 (endothelial cells) or on laminins 521/111 in a ratio 1:3 (hepatic progenitors). RLU = Relative Light Units. Blend: samples from liver spheres composed of differentiated cells that initially were plated and cultured on laminins 521/111 in a ratio of 1:3. 521: samples from liver spheres composed of differentiated cells that were initially plated and cultured on laminin 521 exclusively. Mix: samples from liver spheres composed of differentiated cells that initially were plated and cultured on laminin 521 (endothelial cells) and on laminins 521/111 (hepatic progenitors) in a ratio 1:3.

### Detailed description of the invention

The present invention relates to methods for producing endothelial cells and the cells obtained from the method. The invention further relates to methods of producing liver an aggregation of liver cells comprising the endothelial cells of the present invention.

A more complete understanding of methods of the present invention can be obtained reference to the detailed description of the invention and by reference to the examples and accompanying drawings. The examples and drawings represent working exmples and are therefore not intended to define or limit the scope of the exemplary embodiments.

A stem cell is an undifferentiated cell from which specialized cells are subsequently derived. Pluripotent stem cells can be differentiated into any of the three germ layers: endoderm, mesoderm, or ectoderm. Post fertilization, pluripotent stem cells form every cell type in the human body, including less plastic stem cell populations such as adult stem cells, fetal stem cells, and amniotic stem cells.

Embryonic stem cells are a type of pluripotent stem cell, and possess extensive self-renewal capacity and pluripotency. Another type of pluripotent stem cell, induced pluripotent stem cells, were produced from mammalian terminally differentiated cells by a process termed somatic cell reprogramming. The process by which a stem cell changes into a more specialized cell is referred to as differentiation, for example the differentiation of endodermal progenitor cells to hepatocytes.

The present invention provides methods for producing endothelial cells. The methods uses pluripotent stem cells. In one embodiment, pluripotent stem cells are selected from embryonic stem cells, induced pluripotent stem cells, adult stem cells, fetal stem cells, amniotic stem cells, and any combination thereof. In one embodiment, the pluripotent stem cells are mammalian pluripotent stem cells. In a preferred embodiment, the pluripotent stem cells are human pluripotent stems cells. In a further embodiment, the pluripotent stem cells are human pluripotent stems cells, which have not been obtained by desctruction of human embryos. Human pluripotent stem cells may express one or more of the markers (or all markers) from the list consisting of SSEA-3, SSEA- 4, TRA-1 -60, TRA-1 -81, TRA-2-49/6E, ALP, Sox 2, Ecadherin, UTF-1, Oct4, Lin28, Rex-1, and Nanog.

In *vitro*/*ex vitro* differentiation of stem cells generally includes a substrate or coating that provides a structural support for the cell; and a cell culture medium to provide nutrition to the cell. The substrate or coating is typically formed as a layer in a container, for example a petri dish, in the well of a multi-well plate or flasks such as T flasks, for example T75 or T175 flasks.

The method of the present invention, the cell culture substrate comprises the combination of two laminins, i.e. laminin-521 and laminin-111 (or a functional fragment thereof or one or both of the laminins).

Laminins are a family of heterotrimeric glycoproteins that reside primarily in the basal lamina. They function via binding interactions with neighboring cell receptors on the one side, and by binding to other laminin molecules or other matrix proteins such as collagens, nidogens or proteoglycans. The laminin molecules are also important signaling molecules that can strongly influence cellular behavior and function. Laminins are important in both maintaining cell/tissue phenotype, as well as in promoting cell growth and differentiation in tissue repair and development.

A laminin protein molecule comprises one alpha-chain subunit, one beta-chain subunit, and one gamma-chain subunit, all joined together in a trimer through a coiled-coil domain. There exist five different alpha chains, three beta chains and three gamma chains that in human tissues have been found in at least fifteen different combinations.

In the context of the present invention the term "laminin-521" refers to the protein formed by joining alpha5, beta2 and gamma1 chains together. The term laminin-111 (laminin-1) refers to the polypeptide that contains alpha-1, beta-1 and gamma-1 chains.

In the context of the present invention, reference is made to fragments of laminin-521 and fragments of laminin-111. It should be understood that the recited fragments comprise the domains of the full polypeptide that is essential for the maintaining an activity corresponding essentially to that of the full length laminin.

The laminin may be obtained by recombinant expression in any suitable host cell. Such laminin are referred to as a recombinant laminin. Alternatively, the laminin from naturally sources (non-recombinant).

The method for producing endothelial cells, kits of parts of the present invention typically includes three types of media:
A medium that is used for the initial seeding of the pluripotent cells on the substrate and allowing the cells to expand to reach the preferred cell density. This medium is also referred to herein as the first medium. The first medium is typically a serum-free cell culture medium suitable for the feeder-free maintenance and expansion of pluripotent stem cells, such as mTeSR (MT) Plus (StemCell Technologies).

### Mesoderm priming medium

The mesoderm priming medium is a serum-free medium that allows for differentiating cells to mesoderm lineage, and it typically combines high concentration of glucose, amino acids, and vitamins with F-12's wide variety of components as well as phenol red. The mesoderm priming medium may be 1:1 mixture of DMEM:F12 and CTS Neurobasal medium. The priming medium may be supplemented with GlutaMAX, N2 CTS, B27 CTS, B-mercaptoethanol. As disclosed herein, the mesoderm priming also comprises factor that primes the stem cells into mesoderm, such as CHIR99021 and BMP4.

### Endothelial differentiation medium

Endothelial differentiation medium refers to that induce the differentiation of the stem cells to endothelial cells. Accordingly, endothelial differentiation medium is formulated to support the development of (human) endothelial cells from stem cells. The endothelial differentiation medium is typically StemPro-34 SFM medium supplemented with differentiation factors such as VEGF and forskolin. StemPro^{®}-34 SFM consists of a basal liquid medium and frozen 40X nutrient supplement (StemPro^{®}-34 Nutrient Supplement) which is added at the time of use. StemPro^{®}-34 SFM is manufactured without cytokines and hematopoietic growth factors, giving the investigator the freedom to use any factor or combination of factors required in ones' studies. StemPro^{®}-34 SFM contains components of human, recombinant or synthetic origin and is performance-tested on normal human bone marrow.

In one embodiment, the culture medium used in the method is a serum free medium.

During the preparation of the endothelial cells, the medium is typically renewed with fresh medium for example about every 24 hours.

Hepatic progenitor cells (also known as hepatoblasts) is a fetal precursor of the hepatocyte. In particular, hepatoblasts are parenchymal cells of fetal livers and are capable of self-renewal in culture and differentiation into hepatocytes or biliary epithelium.

In the context of the present invention, endothelial progenitor cells have been typically defined as cells that are able to differentiate into endothelial cells and contribute e.g. to the formation of blood vessels. Endothelial progenitor cells express among other antigens, CD34, CD133, type 2 VEGF receptor (VEGFR-2 or Flk-1), and the CXCR4 chemokine receptor.

In a first aspect, the present invention provides a method for producing endothelial cells, comprising:
seeding pluripotent stem cells on a cell culture substrate comprising, consisting of or essentially consisting of:
   (i) laminin-521 or a fragment of laminin-521; and
   (ii) laminin-111 or a fragment of laminin-111; and
culturing the pluripotent stem cells in the presence of one or more differentiation factors that stimulates endothelial cell differentiation of said pluripotent stem cells to obtain said endothelial cells.

The method for producing endothelial cells typically involves priming pluripotent stem cells towards mesoderm (differentiating the pluripotent stem cells to mesodermal cells). At this stage the cells are typically cultured in mesoderm priming medium as described herein comprising factors that primes pluripotent stem cells towards mesoderm (these cells are also referred to as definitive mesoderm). Factors that may be used to facilitate the transition towards mesoderm typically comprises an activator of ERK, JNK and p38 MAPK signalling pathways and/or an inducer/activator of SMAD signalling pathways. For example, an BMP4 activator/inducer in the form of an agonist that activates canonical BMP signaling and increases SMAD-1/5/9 phosphorylation. One example of an agonist molecules of BMP4 is SB4.

In one embodiment, the CHIR99021 (CHIR), Bone morphogenetic protein 4 (BMP4), or Wnt Family Member 3a (Wnt3a) or any combination thereof is used to prime the pluripotent stem cells towards mesoderm. In a further embodiment, a combination of CHIR99021 (CHIR) and Bone morphogenetic protein 4 (BMP4) is used to prime the pluripotent stem cells towards mesoderm.

The pluripotent stem cells that have been primed towards mesoderm (differentiated to mesodermal cells) may subsequently be differentiated to endothelial cells by culturing the cells in the presence of one or more factors that stimulate the differentiation to endothelial cells. The one or more factors that stimulate the differentiation to endothelial cells include but are not limited to VEGF (Vascular endothelial growth factor), placenta-like growth factor 1 (PLGF-1) and activators adenylate cyclase activator. In one embodiment said small molecule adenylate cyclase activator leads to the activation of PKA/PKI signaling pathway. In one embodiment, said small molecule adenylate activators is selected from the group consisting of Forskolin ((3R)-(6aalphaH)Dodecahydro-6beta,10alpha,10balpha-trihydroxy 3beta,4abeta,7,7,10abeta-pentamethyl-l-oxo-3-vinyl-lH-naphtho[2,l-]pyran-5beta-yl acetate), 8-Bromo-cAMP (8-Bromoadenosine-3',5'-cyclic monophosphate) and Adrenomedullin. In one embodiment, VEGF and a small molecule adenylate cyclase activator selected from the group consisting of forskolin, 8-Bromo-cAMP and Adrenomedullin is used to differentiate the mesodermal cells to endothelial cells. In another embodiment, VEGF and forskolin is used to differentiate the mesodermal cells to endothelial cells. The differentiated cells may be isolated by enriching the population of cells for cells that express endothelial, such as CD31 and CD144.

The inventors have discovered that the endothelial cells obtained by the method of the present by differentiating pluripotent stem cells to endothelial cells on a substrate (matrix) comprising the combination of laminin 521 and laminin 111 are surperior over endothelial cells obtained using a substrate of laminin 521 only. The inventors have discovered that the endothelial cells of the present invention may be used to prepare improved aggregation with other cells types such as an aggregation with hepatic progenitor cells. In particular, the inventors have discovered that the aggregation of endothelial cells of the present invention and hepatic progenitor cells produce larger and healthier cells spheres with no visible necrotic cores (unlike endothelial cells obtained on a matrix of laminin 521 only). (See Example 3 and Figure 4). Accordingly, improved endothelial cells may be obtained by conduting the differentiation of pluripotent stem cells to endothelial cells on a matrix comprising laminin-521 (or a functional fragment of laminin-521) and laminin-111 (or a functional fragment of laminin-111).

In order to comply with good manufacturing practice (GMP), the laminins used by the method of the present invention is isolated laminins, for example lamining obtained by recombinant expression in a suitable host.

Basement membrane extracts such as Matrigel, Cultrex and Geltrex comprise laminins. However, these products are commonly occurring lot-to-lot variability during manufacturing and complexity in composition, which are often poorly defined, making it difficult to determine exactly which signals are promoting cell function. Thus, the use of Matrigel, Cultrex and Geltrex (animal-derived matrix) do not comply with good manufacturing practice (GMP) and therefore none of these products used in the method of the present invention. In one embodiment of the present invention, the methods of the invention compatible with good manufacturing practice (GMP). Thus, the cells or cell aggregates obtained by the methods compatible with good manufacturing practice (GMP) are void of unknown factors that render the cell and tissues incompatible with subsequent clinical applications.

In a preferred embodiment, the laminins used by the method of the present invention are human laminins, such as human laminin 521 and human laminin 111. In another preferred embodiment, the laminins including human laminin 521 and human laminin 111 are obtained by recombinant expression. A laminin obtained by recombinant expression is reffered to as a recombinant laminin. The cell culture substrate on which the pluripotent stem cells are seeded may be a matrix consisting of or essentially consisting of laminin 521 and laminin 111 (or functional fragments thereof), for example human laminin 521 and human laminin 111. In a further embodiment, the cell culture substrate comprises laminin 521 and laminin 111 (or functional fragments thereof), such as recombinant human laminin 521 and human laminin 111. In a further embodiment, the cell culture substrate comprises laminin 521 and laminin 111 and one or more additional components, for example matrix proteins, such as collagen I and fibronectin.

Preferably, the polypeptide components of the cell culture substrate including laminin 521 and laminin 111 are recombinant polypeptides. In the context of the present invention, the term "recombinant" polypeptide refers to a polypeptide, which is produced by expression from an exogeneous polynucleotide encoding the polypeptide in a suitable host cell.

The method for producing endothelial cells, comprises seeding pluripotent stem cells on a cell culture substrate comprising, consisting of or essentially consisting of laminin-521 (or a fragment of laminin-521) and laminin-111 (or a fragment of laminin-111). Preferably, the laminin-521 and laminin-111 are human laminins such as recombinant human laminins.

In one embodiment, the concentration of laminin-521 and laminin-111 in said cell culture substrate is in the range of about 1 to 10 microgram(s)/ml, for example 3 to 8 microgram(s)/ml, such as 4 to 7 microgram(s)/ml, for example 5 to 6 microgram(s)/ml, such as about 5 ug/ml, such as 1.25 µg laminin-521/ml and 3.75 µg laminin-111/ml.

The above amount of laminin may also expressed as microgram(s)/cm2. Thus, in one embodiment, the concentration of laminin-521 and laminin-111 on said cell culture substrate is 1 microgram(s)/10cm² - 10 microgram(s)/10cm², for example 3 microgram(s)/10cm² - 8 microgram(s)/10cm², such as 4 microgram(s)/10cm² - 7 microgram(s)/10cm², for example 5 to 6 microgram(s)/10cm², such as about 5 microgram(s)/10cm², such as 1.25 microgram(s) laminin-521/10cm² and 3.75 microgram(s) laminin-111/10cm².

In another embodiment, the concentration by weight of laminin-111 in said cell culture substrate is equal to or higher than the concentration of laminin-521, such as the ratio of laminin-521 to laminin-111 is in the range of 1:1 to 1:4, such as about 1:3. In one embodiment, the ratio of laminin-521 to laminin-111 (by weight) is about 1:3.

The pluripotent stem cells are typically seeded on said culture substrate at a density of 25000 to 40000 pluripotent stem cells per cm², such as 30000 to 35000 pluripotent stem cells per cm², for example 30000 pluripotent stem cells per cm². The inventors have discovered that the optimal seeding density of the pluripotent stem cells is about 30000 pluripotent stem cells per cm². (See Figure 3) Thus, in one embodiment, the pluripotent stem cells are seeded at a density of about 30000 pluripotent stem cells per cm².

After the pluripotent stem cells have been seeded on the culture substrate, the cells are allowed to expand by proliferation to reach a confluency in the range of about 30 to 60%, such as 30 to 55% such as 30 to 50%, for example 40 to 50%, such as about 40% before contacting the cells with differentiation factors. Thus in one embodiment, said one or more differentiation factors is added when the cells have reached a confluency in the range of about 30 to 60%, such as 30 to 55% such as 30 to 50%, for example 40 to 50%, such as about 40%. In another embodiment, said one or more differentiation factors is added when the cells have reached a confluency about 40%.

The one or more differentiation factor is typically added after seeding the pluripotent stem cells on a cell culture substrate. In one embodiment, said one or more differentiation factors is added to the cells about 12 to 96 h after seeding the pluripotent stem cells on a cell culture substrate, such as 24 to 96 h after seeding the pluripotent stem cells on a cell culture substrate, for example 24 to 72 h after seeding the pluripotent stem cells on a cell culture substrate, such as about 24 h and/or 72 h after seeding the pluripotent stem cells on a cell culture substrate.

As described herein, in a first step, the pluripotent stem cells are typically primed towards mesoderm (differentiating the pluripotent stem cells to mesodermal cells). In further step, mesodermal cells obtained from the pluripotent stem cells are contacted with diffentiation factors that stimulate the differentiation of the mesodermal cells to endothelial cells. Thus, in one step the pluripotent stem cells are typically contacted with one or more diffentiation factors that stimulates differentiation of the pluripotent stem cells to mesodermal cells (e.g. using the mesoderm priming medium described herein). Subsequently, the medium is typically replaced with one or more diffentiation factors that stimulates differentiation to endothelial cells (e.g. using the mesoderm differentiaion medium described herein).

In another embodiment, said one or more differentiation factors (stimulating the differentiation of pluripotent cells to mesodermal cells) is added to the cells about 12 to 48 h after seeding the pluripotent stem cells and further one or more differentiation factors (stimulating the differentiation of the mesodermal cells to endothelial cells) is added 72 to 96 h after seeding the pluripotent stem cells, such as one or more differentiation factors is added about 24 h and further one or more differentiation factors is added 72 h after seeding the pluripotent stem cells on a cell culture substrate.

In one embodiment, the one or more factors that stimulates endothelial cell differentiation of said pluripotent stem cells is an agonist of Wnt signalling, such as by antagonizing GSK3-signalling.In one embodiment, the one or more factors that stimulates endothelial cell differentiation of said pluripotent stem cells is an antagonist of GSK3b.

In one embodiment, the one or more factors that stimulates endothelial cell differentiation of said pluripotent stem cells is selected from the group consisting of CHIR99021 (CHIR), Bone morphogenetic protein 4 (BMP4), Vascular endothelial growth factor (VEGF), forskolin and Wnt3a.

As described herein, CHIR99021 (CHIR) and/or Bone morphogenetic protein 4 (BMP4) is typically used to stimulate the differentiation of pluripotent stem cells to mesodermal cells. Thus, in one embodiment, CHIR99021 (CHIR) and/or Bone morphogenetic protein 4 (BMP4) is added to the cells about 12 to 48 h after seeding the pluripotent stem cells on a cell culture substrate, such as 24 to 48 h after seeding the pluripotent stem cells on a cell culture substrate, for example about 24 h after seeding the pluripotent stem cells on a cell culture substrate.

In one embodiment, CHIR99021 (CHIR) is added as a culture medium comprising CHIR99021 (CHIR) within the range of 7 to 10 µM CHIR99021 (CHIR), such as 7 to 9 µM CHIR99021, for example 8 to 9 µM CHIR99021, such as about 8 µM CHIR99021. In another embodiment, CHIR99021 (CHIR) is added as a culture medium comprising about 8 µM CHIR99021 per ml medium.

In one embodiment, Bone morphogenetic protein 4 (BMP4) is added as a culture medium comprising Bone morphogenetic protein 4 (BMP4) at concentration within the range of 15 - 35 ng Bone morphogenetic protein 4 (BMP4) per ml medium, such as 25 - 30 ng Bone morphogenetic protein 4 (BMP4) per ml medium, such as about 25 ng Bone morphogenetic protein 4 (BMP4) per ml medium. In another embodiment, Bone morphogenetic protein 4 (BMP4) is added as a culture medium comprising about 25 ng Bone morphogenetic protein 4 (BMP4) per ml medium.

The pluripotent stem cells typically cultured in the presence of the one or more differentiation factors for a period of 24 h to 96 h, such as 48 h to 96 h, for example 72 h to 96 h, such as about 72 h. For example, the step of differentiating the cells to mesodermal cells may be carried out by contacting the cells with one or more differentiation factors for 48 h to 96 h, for example 72 h to 96 h, such as about 72 h. The subsequent step of differentiating the cells to endothelial cells may be carried out by contacting the cells with one or more differentiation factors for 24 h to 48 h, such as about 48 h.

The culture medium is typically renew during the differentiation process. For example, the culture medium may be renewed every 24 h or at least once during the differentiation process.

In one embodiment, the Vascular endothelial growth factor (VEGF) and/or forskolin is added to the cells about 48 to 96 h after seeding the pluripotent stem cells, such as 48 to 72 h after seeding the pluripotent stem cells, for example about 72 h after seeding the pluripotent stem cells.

In another embodiment, the Vascular endothelial growth factor (VEGF) is added as culture medium comprising Vascular endothelial growth factor (VEGF) at a concentration within a range of 100 to 300 ng/ml, such as 150 to 250 ng/ml, such as about 200 ng Vascular endothelial growth factor (VEGF)/ml.

In a further embodiment, forskolin is added as a culture medium comprising forskolin at a concentration within the range of 1 to 3 µM, such as about 2 µM.

The method typically comprises two steps of differentiation: a first step introducing differentiation factors (such as CHIR99021 and/or BMP4) that induce differentiation to mesodermal cells and a subsequent step that introducing differentiation factors (such as VEGF and/or forskolin) that induce differentiation to endothelial cells. Typically, the medium comprising the differentiation factors for mesodermal priming is replaced with the medium comprising the differentiation factors for endothelial differentiation.

In one embodiment, the culture medium comprising Vascular endothelial growth factor (VEGF) and/or forskolin replaces the culture medium comprising CHIR99021 (CHIR) and/or Bone morphogenetic protein 4 (BMP4).

In one embodiment, method for producing endothelial cells, comprises:
seeding pluripotent stem cells on a cell culture substrate comprising, consisting of or essentially consisting of: (i) laminin-521 or a fragment of laminin-521; and
   (ii) laminin-111 or a fragment of laminin-111; and
culturing the pluripotent stem cells in a first culture medium for about 24 hours or until the confluency of the cells are in the range of about 30 to 60%, such as 30 to 55% such as 30 to 50%, for example 40 to 50%, such as about 40%.;
replacing the first culture medium with a second culture medium (mesoderm priming medium) comprising CHIR99021 (CHIR) and Bone morphogenetic protein 4 (BMP4) and culture the cells for about 72 hours;
replacing the second culture medium comprising with a third culture medium (endothelial differentiation medium) comprsing VEGF and forskolin and culture the cells for about 48 hours.

In another embodiment, the concentration of laminin-521 and laminin-111 in said cell culture substrate is about 5 ug/ml, such as 1.25 µg laminin-521/ml and 3.75 µg laminin-111/ml. In a further embodiment, the concentration by weight of laminin-111 in said cell culture substrate is equal to or higher than the concentration of laminin-521, such as the ratio of laminin-521 to laminin-111 is in the range of 1:1 to 1:4, such as about 1:3. In one embodiment, the concentration by weight of laminin-521 to laminin-111 is about 1:3.

In one embodiment, the second culture medium (mesoderm priming medium) comprises CHIR99021 (CHIR) within the range of 7 to 10 µM CHIR99021 (CHIR), such as 7 to 9 µM CHIR99021, for example 8 to 9 µM CHIR99021, such as about 8 µM CHIR99021. In one embodiment, the concentration of CHIR99021 in the second culture medium (mesoderm priming medium) is about 8 µM.

In one embodiment, second culture medium (mesoderm priming medium) comprises Bone morphogenetic protein 4 (BMP4) at concentration within the range of 15 - 35 ng Bone morphogenetic protein 4 (BMP4) per ml medium, such as 25 - 30 ng Bone morphogenetic protein 4 (BMP4) per ml medium, such as about 25 ng Bone morphogenetic protein 4 (BMP4) per ml medium. In another embodiment, the concentration of Bone morphogenetic protein 4 (BMP4) in the second culture medium is about 25 ng Bone morphogenetic protein 4 (BMP4) per ml medium.

In one embodiment, second culture medium (mesoderm priming medium) comprises CHIR99021 (CHIR) and Bone morphogenetic protein 4 (BMP4) such as about 8 µM CHIR99021 and about 25 ng Bone morphogenetic protein 4 (BMP4) per ml medium.

In one embodiment, the third culture medium (endothelial differentiation medium) comprises Vascular endothelial growth factor (VEGF) at a concentration within a range of 100 to 300 ng/ml, such as 150 to 250 ng/ml, such as about 200 ng Vascular endothelial growth factor (VEGF)/ml. In another embodiment, the concentration of Vascular endothelial growth factor (VEGF) is about 200 ng Vascular endothelial growth factor (VEGF)/ml.

In one embodiment, the third culture medium (endothelial differentiation medium) comprises forskolin at a concentration within the range of 1 to 3 µM, such as about 2 µM. In another embodiment, the concentration of forskolin is about 2 µM.

In one embodiment, the third culture medium (endothelial differentiation medium) comprises Vascular endothelial growth factor (VEGF) at a concentration of about 200 ng Vascular endothelial growth factor (VEGF)/ml and forskolin at a concentration of about 2 µM.

The differentiation of the pluripotent stem cells to endothelial cells is characterized by markers, which may be used to identify and isolate the endothelial cells from a population of cells comprising endothelial cells and cells, which are not differentiated. Immunocytochemistry, flow cytometry, and enzymatic analysis and RT-PCR are examples of methods for analysis of the cell population. MACS or FACS sorting are examples of methods that may be used to isolate endothelial cells from the population of cells.

Markers for endothelial cells includes VE-cadherin (VE-CADH, CD144) and cluster of differentiation 31 (CD31).

In one embodiment, about 0% of the endothelial cells obtained by the method are negative for Alpha-fetoprotein (AFP) markers. In another embodiment, the endothelial cells are positive for at least one marker selected from the list consisting of: VE-cadherin (VE-CADH, CD144) positive, CD31 positive and the combination of VE-cadherin (VE-CADH, CD144) and CD31 positive.

In one embodiment, the method comprises a further step of isolating VE-cadherin (VE-CADH, CD144) positive and/or CD31 positive cells, such as by MACS or FACS sorting, such as by isolating VE-cadherin (VE-CADH, CD144) positive cells.

In another embodiment, the cells obtained by the method comprises at least 20% VE-cadherin (VE-CADH, CD144) positive endothelial cells, such as at least 30% VE-cadherin (VE-CADH, CD144) positive endothelial cells, for example at least 40% VE-cadherin (VE-CADH, CD144) positive endothelial cells, such as at least 50% VE-cadherin (VE-CADH, CD144) positive endothelial cells, for example at least 60% VE-cadherin (VE-CADH, CD144) positive endothelial cells, such as at least 70% VE-cadherin (VE-CADH, CD144) positive endothelial cells, such as 20 to 40% VE-cadherin (VE-CADH, CD144) positive endothelial cells, for example 20 to 70% VE-cadherin (VE-CADH, CD144) positive endothelial cells, such as 40 to 70% VE-cadherin (VE-CADH, CD144) positive endothelial cells, for example 50 to 70% VE-cadherin (VE-CADH, CD144) positive endothelial cells, such as 60 to 70% VE-cadherin (VE-CADH, CD144) positive endothelial cells.

Endothelial cells are also characterized by being negative for the Alpha-fetoprotein (AFP) marker.

In one embodiment, the cells obtained by the method comprises at least 20% cluster of differentiation 31 (CD31) positive endothelial cells, such as at least 30% cluster of differentiation 31 (CD31) positive endothelial cells, for example at least 40% cluster of differentiation 31 (CD31) positive endothelial cells, such as at least 50% cluster of differentiation 31 (CD31) positive endothelial cells, for example at least 60% cluster of differentiation 31 (CD31) positive endothelial cells, such as at least 70% cluster of differentiation 31 (CD31) positive endothelial cells, for example at least 80% cluster of differentiation 31 (CD31) positive endothelial cells, such as 20 to 80% cluster of differentiation 31 (CD31) positive endothelial cells.

In a further aspect, the present invention provides endothelial cells obtained by the method according to the invention. As described herein, the inventors have discovered that the endothelial cells obtained by the method according to the invention are superior over endothelial cells produced on a support based on laminin 521 only. The endothelial cells of the present invention may be used to prepare cells aggregates with other cells types such as hepatic progenitor cells.

Thus in one aspect, the present invention provides a composition comprising the endothelial cells obtained by the method of the present invention and cells of a further cell type, such as hepatic progenitor cells or progenitor cells of endodermal, mesodermal and ectodermal origin. In one embodiment, the composition comprises hepatic progenitor cells and the endothelial cells of the present invention.

In a further aspect, the present invention provides a kit of parts comprising:
(i) a cell culture substrate comprising, consisting of or essentially consisting of: laminin-521 or a fragment of laminin-521 and laminin-111 or a fragment of laminin-111; and
(ii) at least one culture medium comprising one or more differentiation factors that stimulates endothelial cell differentiation of said pluripotent stem cells.

The kit of parts may comprise separate containers of culture medium, such as a container with culture medium for mesodermal priming and a container with culture medium for endothelial cell differentiation. The differentiation factors may be provided in separate containers or included in the culture medium provided with the kit. The kit may include one or more containers for culturing the stem cells, e.g petri-dishes, multi-well plates, T flasks such as T75 or T175 flasks or the like. The container may be pre-coated with laminin-521 and laminin-111. Alternatively, the laminin-521 and laminin-111 may be provided with the kit for coating the container(s) with a cell culture substrate comprising said laminins.

In one embodiment, the concentration of laminin-521 and laminin-111 in said cell culture substrate is in the range of about 1 to 10 microgram(s)/ml, for example about 3 to 8 microgram(s)/ml, such as 4 to 7 microgram(s)/ml, for example 5 to 6 microgram(s)/ml, such as about 5 ug/ml, such as 1.25 µg laminin-521/ml and 3.75 µg laminin-111/ml. Thus, containers provided may be pre-coated with the laminins to reach the recited concentration or the laminins are provided in suitable amounts for coating of the container(s).

In another embodiment, the concentration of laminin-111 in said cell culture substrate is equal to or higher than the concentration of laminin-521, such as the ratio of laminin-521 to laminin-111 is in the range of 1:1 to 1:4, such as about 1:3.

In a further embodiment, the kit comprises at least one culture medium comprising CHIR99021 (CHIR) and/or Bone morphogenetic protein 4 (BMP4).

In one embodiment, the kit comprises at least one culture medium comprising CHIR99021 (CHIR) within the range of 7 to 10 µM CHIR99021 (CHIR), such as 7 to 9 µM CHIR99021, for example 8 to 9 µM CHIR99021, such as about 8 µM CHIR99021. Alternatively, the kit is provided with CHIR99021 (CHIR) to be supplemented in at least one culture medium to reach the recited concentration.

In another embodiment, the kit comprises at least one culture medium comprising Bone morphogenetic protein 4 (BMP4) at concentration within the range of 15 - 35 ng per ml medium, such as 25 - 30 ng Bone morphogenetic protein 4 (BMP4) per ml medium, such as about 25 ng Bone morphogenetic protein 4 (BMP4) per ml medium.

In one embodiment, the kit comprises at least one culture medium comprising Vascular endothelial growth factor (VEGF) and forskolin.

In one embodiment, the kit comprises at least one culture medium comprising Vascular endothelial growth factor (VEGF) at a concentration within a range of 100 to 300 ng/ml, such as 150 to 250 ng/ml, such as about 200 ng Vascular endothelial growth factor (VEGF)/ml.

In another embodiment, the kit comprises at least one culture medium comprising forskolin at a concentration within a range of 1 to 3 µM, such as about 2 µM.

In one embodiment, the kit comprises:
a culture medium comprising CHIR99021 (CHIR) and Bone morphogenetic protein 4 (BMP4), and
a further culture medium comprising Vascular endothelial growth factor (VEGF) and forskolin.

In a further embodiment, said culture medium comprises about 8 µM CHIR99021 and about 25 ng Bone morphogenetic protein 4 (BMP4). For example, a 1:1 mixture of DMEM:F12 and CTS Neurobasal medium comprising CHIR99021 (CHIR) and Bone morphogenetic protein 4 (BMP4), such as about 8 µM CHIR99021 and about 25 ng Bone morphogenetic protein 4 (BMP4).

In yet a further embodiment, said further culture medium comprises about 200 ng Vascular endothelial growth factor (VEGF)/ml and about 2 µM forskolin. For example, StemPro-34^{™} comprising Vascular endothelial growth factor (VEGF) and forskolin, such as about 200 ng Vascular endothelial growth factor (VEGF)/ml and about 2 µM forskolin.

In one embodiment, the concentration of laminin-521 and laminin-111 in said cell culture substrate is about 5 ug/ml, such as 1.25 µg laminin-521/ml and 3.75 µg laminin-111/ml. In another embodiment, the ratio of laminin-521 to laminin 111 in said cell culture substrate is about 1:3.

In a further embodiment, at least one of the culture medium is a serum free medium.

In a further aspect, the present invention provides the use of the cells obtained by the method of the present invention or the composition comprising the endothelial cells for the preparation of an aggretation of cells. In one embodiment, the aggregation is an aggregation of endothelial cells and hepatocytes. Thus, in the process of aggregation, the hepatic progenitors of the mixture of hepatic progenitors and endothelial cells matures to become hepatocytes.

The described herein the inventors have discovered that the endothelial cells obtained by the method of present invention are useful for producing an aggregation of liver cells. In particular, the inventors have discovered that the cells obtained using the blend of laminin 521 and laminin 111 produces endothelial cells that facilitate the production of liver cells aggregates characterized in that the aggregates have a healthier appearance and do not contain visible necrotic cores. (See Example 3, Figure 4). The use of the blend of laminin 521 and laminin 111 further produces endothelial cells that in the process of aggregation of liver cells generates aggregates that display a more consistent and decreasing AFP secretion (marker for foetal liver) over time relative to endothelial cells prepared on a coating of laminin 521 only. (See Example 3, Figure 5). Further, the cells obtained using the blend of laminin 521 and laminin 111 further produces endothelial cells that in the process of aggregation of liver cells generates aggregates that display consistent secretion of albumin (ALB) as a marker for liver maturation. (See Example 3, Figure 6).

The present invention provides a method for producing an aggregation of liver cells, said method comprising the step of:
(i) providing endothelial cells obtained by the method according to the present invention described herein;
(i) providing hepatic progenitor cells;
(ii) prepare a mixture comprising said hepatic progenitor cells and endothelial cells in a culture medium;
(iii) seed the mixture on a cell culture substrate;
(iv) allow the mixture of cells to propagate and form an aggregation of cells;
(v) collect said aggregation of cells.

In a preferred embodiment, the endothelial cells and the hepatic progenitor cells are human cells.

In one embodiment, the ratio of hepatic progenitor cells to endothelial cells in the mixture is such that the number of hepatic progenitor cells is equal to or higher than the number of endothelial cells. In one embodiment, the ratio of hepatic progenitor cells to endothelial cells in the mixture is in the range of 1:1 to 4:1, such as 1:1 to 3.5:1, for example 1:1 to 3:1, such as 1:1 to 2:1. In one embodiment, the ratio of hepatic progenitor cells to endothelial cells in the mixture is 3:1. In another embodiment, the ratio of hepatic progenitor cells to endothelial cells in the mixture is 3.3:1.

In another embodiment, the endothelial cells have been enriched for VE-cadherin (VE-CADH, CD144) positive cells. For examples, such that at least 20% of the endothelial cells are VE-cadherin (VE-CADH, CD144) positive, such as at least 25% of the endothelial cells are VE-cadherin (VE-CADH, CD144) positive, for example at least 30% of the endothelial cells are VE-cadherin (VE-CADH, CD144) positive, such as 20 to 40% of the endothelial cells VE-cadherin (VE-CADH, CD144) positive.

In one embodiment, the hepatic progenitor cells are obtained by differentiating pluripotent stem cells, preferably human pluripotent stem cells, to hepatic progenitor cells by culturing the pluripotent stem cells on a cell culture substrate comprising laminin 521 and laminin 111 (or functional fragments of thereof) and contacting the cells with differentiation factors that induce the hepatic differentiation.

In another embodiment, 90-100% of the hepatic progenitor cells are positive for AFP and 90-100% of the hepatic progenitor cells are positive for HNF4a, such as 95-100% positive for AFP and 95-100% positive for HNF4a. In the process of aggregation the hepatic progenitor cells will mature and the AFP marker (foetal marker) will descrease, which markers for maturation will appear such that the hepatocytes in the aggregation typically will be positive for Albumin, HNF4a, CYP1A2 and A1AT. In one embodiment, the liver cells of the cells aggregate produced by the method are positive for HNF4a and display CYP1A2 function and albumin and A1AT secretion.

In one embodiment, the hepatic progenitor cells used by the method of the present invention have been obtained by differentiating pluripotent stem cells, preferably human pluripotent stem cells, to hepatic progenitor cells by culturing the pluripotent stem cells on a cell culture substrate comprising laminin 521 and laminin 111 (or functional fragments of thereof) and contacting the cells with differentiation factors that induce the hepatic differentiation.

In another embodiment, the hepatic progenitor cells have been obtained by seeding pluripotent stem cells on a cell culture substrate comprising a mixture of laminin-521 or a fragment thereof and laminin-111 or a fragment thereof; and culturing the pluripotent stem cells to obtain said hepatic progenitor cells. In one embodiment, the weight ratio of laminin-111 to laminin-521 is from about 4:1 to about 1:1.

In one embodiment, the hepatic progenitor cells used by the method of the present invention have been obtained by culturing the pluripotent stem cells in the presence of activin A and Wnt3a. In a further embodiment, the hepatic progenitor cells have been obtained by culturing the pluripotent stem cells in the presence of activin A and Wnt3a followed by culturing the pluripotent stem cells in the presence of hydrocortisone (HC), hepatocyte growth factor (HGF), and oncostatin m (OSM).

In one embodiment, the culture medium comprises 10 µM Y-27632 (ROCKi), 50 ng/ml VEGF (1:200), 10 ng/ml EGF (1:1000), 10 ng/ml FGF (1:1000), 10 ng/ml HGF (1:1000) and 20 ng/ml OSM (1:1000) (final amounts in the culture medium).

The cell culture substrate used by the method is typically a mould such as an agarose mould, which may be produced as described in Example 3.

In one embodiment, the mixture comprising the hepatic progenitor cells and endothelial cells is seeded on the center of said mould. In a further embodiment, the mixture of cells are allowed to propagate until the volume of cells exceeds the volume of the mould and subsequently transferring the cells to a well comprising culture medium, such as a mixture of William's and SFM endothelial media, for example a 1:1 mixture of William's and SFM endothelial media.

As described above, the mixture of cells are typically allowed to propagate in the mould until the volume of cells exceeds the volume of the mould before transferring the cells to a well (e.g. of a multi-well plate) comprising culture medium. The period to reach the preferred volume of cells may vary. In one embodiment, the mixture comprising said hepatic progenitor cells and endothelial cells are allowed to propagate for about 5 to 8 days, such as about 7 days and subsequently transferring the cells to a well comprising culture medium for further propagation. During the further propagation the hepatic progenitor cells will mature to become hepatocytes.

In one embodiment, the well is coated with 2-hydroxyethyl methacrylate (poly-HEMA).

In one aspect, the present invention provides an aggregation of liver cells obtained by the method of the present invention. In one embodiment, the liver cells are positive for HNF4a and display CYP1A2 function and albumin and A1AT secretion.

In a further aspect of the present invention, the aggregation of liver cells obtained by the method of the present invention is for use as a medicament.

In some embodiment, the aggregation of liver cells obtained by the method of the present invention is for use in the treatment acute liver failure, chronic liver failure, prepatation for liver implantation, tyrosinemia type I or Alpha-1 antitrypsin deficiency (A1AD or AATD). In one embodiment, the aggregation of liver cells is for use in the treatment acute liver failure, chronic liver failure, or preparation for liver implantation.

The present invention further provides a method for treating, aveliating, reducing, slowing down and/or preventing acute liver failure, chronic liver failure, prepatation for liver implantation, tyrosinemia type I or Alpha-1 antitrypsin deficiency (A1AD or AATD) in a subject, said method comprising administrating a therapeutic effective amount of the aggregation of liver cells of the present invention.

The present invention is further characterized in the following non-limiting embodiments:
Embodiment 1. A method for producing endothelial cells, comprising:
   seeding pluripotent stem cells on a cell culture substrate comprising:
      (i) laminin-521 or a fragment of laminin-521;
      (ii) laminin-111 or a fragment of laminin-111; and
   culturing the pluripotent stem cells in the presence of one or more differentiation factors that stimulates endothelial cell differentiation of said pluripotent stem cells to obtain said endothelial cells.
Embodiment 2. The method according to embodiment 1, wherein the concentration of laminin-521 and laminin-111 in said cell culture substrate is in the range of about 1 to 10 microgram(s)/ml, for example 3 to 8 microgram(s)/ml, such as 4 to 7 microgram(s)/ml, for example 5 to 6 microgram(s)/ml, such as about 5 ug/ml, such as 1.25 µg laminin-521/ml and 3.75 µg laminin-111/ml.
Embodiment 3. The method according to embodiments 1 or 2, wherein the concentration by weight of laminin-111 in said cell culture substrate is equal to or higher than the concentration of laminin-521, such as the ratio of laminin-521 to laminin-111 is in the range of 1:1 to 1:4, such as about 1:3.
Embodiment 4. The method according to any of the preceding embodiments, wherein said one or more factors that stimulates endothelial cell differentiation of said pluripotent stem cells is selected from the group consisting of CHIR99021 (CHIR), Bone morphogenetic protein 4 (BMP4), Vascular endothelial growth factor (VEGF) forskolin and Wnt3a.
Embodiment 5. The method according to any of the preceding embodiments, wherein said one or more differentiation factor is introduced after seeding the pluripotent stem cells on a cell culture substrate.
Embodiment 6. The method according to any of the preceding embodiments, wherein said one or more differentiation factors is added to the cells about 12 to 96 h after seeding the pluripotent stem cells on a cell culture substrate, such as 24 to 96 h after seeding the pluripotent stem cells on a cell culture substrate, for example 24 to 72 h after seeding the pluripotent stem cells on a cell culture substrate, such as about 24 h and/or 72 h after seeding the pluripotent stem cells on a cell culture substrate.
Embodiment 7. The method according to any of the preceding embodiments, wherein said one or more differentiation factors is added to the cells about 12 to 48 h after seeding the pluripotent stem cells and further one or more differentiation factors is added 72 to 96 h after seeding the pluripotent stem cells, such as one or more differentiation factors is added about 24 h and further one or more differentiation factors is added 72 h after seeding the pluripotent stem cells on a cell culture substrate.
Embodiment 8. The method according to any of the preceding embodiments, wherein CHIR99021 (CHIR) and/or Bone morphogenetic protein 4 (BMP4) is added to the cells about 12 to 48 h after seeding the pluripotent stem cells on a cell culture substrate, such as 24 to 48 h after seeding the pluripotent stem cells on a cell culture substrate, for example about 24 h after seeding the pluripotent stem cells on a cell culture substrate.
Embodiment 9. The method according to any of the preceding embodiments, wherein said CHIR99021 (CHIR) is added as a culture medium comprising CHIR99021 (CHIR) within the range of 7 to 10 µM CHIR99021 (CHIR), such as 7 to 9 µM CHIR99021, for example 8 to 9 µM CHIR99021, such as about 8 µM CHIR99021.
Embodiment 10. The method according to any of the preceding embodiments, wherein said Bone morphogenetic protein 4 (BMP4) is added as a culture medium comprising Bone morphogenetic protein 4 (BMP4) at concentration within the range of 15 - 35 ng Bone morphogenetic protein 4 (BMP4) per ml medium, such as 25 - 30 ng Bone morphogenetic protein 4 (BMP4) per ml medium, such as about 25 ng Bone morphogenetic protein 4 (BMP4) per ml medium.
Embodiment 11. The method according to any of the preceding embodiments, wherein cells are cultured in the presence of said one or more differentiation factors for a period of 24 h to 96 h, such as 48 h to 96 h, for example 72 h to 96 h, such as about 72 h.
Embodiment 12. The method according to any of the preceding embodiments, wherein the cells are cultured in culture medium that is renewed after about 24 h after introducing one or more differentiation factors.
Embodiment 13. The method according to any of the preceding embodiments, wherein Vascular endothelial growth factor (VEGF) and/or forskolin is added to the cells about 48 to 96 h after seeding the pluripotent stem cells, such as 48 to 72 h after seeding the pluripotent stem cells, for example about 72 h after seeding the pluripotent stem cells.
Embodiment 14. The method according to any of the preceding embodiments, wherein said Vascular endothelial growth factor (VEGF) is added as culture medium comprising Vascular endothelial growth factor (VEGF) at a concentration within a range of 100 to 300 ng/ml, such as 150 to 250 ng/ml, such as about 200 ng Vascular endothelial growth factor (VEGF)/ml.
Embodiment 15. The method according to any of the preceding embodiments, wherein said forskolin is added as a culture medium comprising forskolin at a concentration within the range of 1 to 3 µM, such as about 2 µM.
Embodiment 16. The method according to any one of embodiments 13 to 15, wherein culture medium comprising Vascular endothelial growth factor (VEGF) and/or forskolin replaces the culture medium comprising CHIR99021 (CHIR) and/or Bone morphogenetic protein 4 (BMP4).
Embodiment 17. The method according to any of the preceding embodiments, wherein the pluripotent stem cells are seeded on said culture substrate at a density of 25000 to 40000 pluripotent stem cells per cm², such as 30000 to 35000 pluripotent stem cells per cm², for example 30000 pluripotent stem cells per cm².
Embodiment 18. The method according to any of the preceding embodiments, said one or more differentiation factors is added when the cells have reached a confluency in the range of about 30 to 60%, such as 30 to 55% such as 30 to 50%, for example 40 to 50%, such as about 40%.
Embodiment 19. The method according to any of the preceding embodiments, said method comprising:
   seeding pluripotent stem cells on a cell culture substrate comprising: (i) laminin-521 or a fragment of laminin-521;
      (ii) laminin-111 or a fragment of laminin-111; and
   culturing the pluripotent stem cells in a first culture medium for about 24 hours or until the cells are about 40% confluent;
   replacing the first culture medium with a second culture medium comprising CHIR99021 (CHIR) and Bone morphogenetic protein 4 (BMP4) and culture the cells for about 72 hours;
   replacing the second culture medium comprising with a third culture medium comprsing VEGF and forskolin and culture the cells for about 48 hours.
Embodiment 20. The method according to embodiment 19, wherein the concentration of laminin-521 and laminin-111 in said cell culture substrate is about 5 ug/ml, such as 1.25 µg laminin-521/ml and 3.75 µg laminin-111/ml.
Embodiment 21. The method according to any of the embodiments 19 and 20, wherein the concentration by weight of laminin-111 in said cell culture substrate is equal to or higher than the concentration of laminin-521, such as the ratio of laminin-521 to laminin-111 is in the range of 1:1 to 1:4, such as about 1:3.
Embodiment 22. The method according to any of the embodiments 19 to 21, wherein said second culture medium comprises CHIR99021 (CHIR) within the range of 7 to 10 µM CHIR99021 (CHIR), such as 7 to 9 µM CHIR99021, for example 8 to 9 µM CHIR99021, such as about 8 µM CHIR99021.
Embodiment 23. The method according to any of the embodiments 19 to 22, wherein said second culture medium comprises Bone morphogenetic protein 4 (BMP4) at concentration within the range of 15 - 35 ng Bone morphogenetic protein 4 (BMP4) per ml medium, such as 25 - 30 ng Bone morphogenetic protein 4 (BMP4) per ml medium, such as about 25 ng Bone morphogenetic protein 4 (BMP4) per ml medium.
Embodiment 24. The method according to any of the embodiments 19 to 23, said second culture medium comprises CHIR99021 (CHIR) and Bone morphogenetic protein 4 (BMP4) such as about 8 µM CHIR99021 and about 25 ng Bone morphogenetic protein 4 (BMP4) per ml medium.
Embodiment 25. The method according to any of the embodiments 19 to 24, wherein said third culture medium comprises Vascular endothelial growth factor (VEGF) at a concentration within a range of 100 to 300 ng/ml, such as 150 to 250 ng/ml, such as about 200 ng Vascular endothelial growth factor (VEGF)/ml.
Embodiment 26. The method according to any of the embodiments 19 to 25, wherein said third culture medium comprises forskolin at a concentration within the range of 1 to 3 µM, such as about 2 µM.
Embodiment 27. The method according to any of the embodiments 19 to 26, wherein said third culture medium comprises Vascular endothelial growth factor (VEGF) at a concentration of about 200 ng Vascular endothelial growth factor (VEGF)/ml and forskolin at a concentration of about 2 µM.
Embodiment 28. The method according to any of the preceding embodiments, wherein said pluripotent stem cells is selected from embryonic stem cells, induced pluripotent stem cells, adult stem cells, fetal stem cells, amniotic stem cells, and any combination thereof.
Embodiment 29. The method according to any of the preceding embodiments, wherein said pluripotent stem cells are human pluripotent stem cells.
Embodiment 30. The method according to any of the preceding embodiments, wherein at least 20% of the endothelial cells obtained by the method are VE-cadherin (VE-CADH) positive, such as at least 25% of the endothelial cells obtained by the method are VE-cadherin (VE-CADH, CD144) positive, such as at least 30% of the endothelial cells obtained by the method are VE-CADH positive, for example at least 40% of the endothelial cells obtained by the method are VE-CADH positive, such as at least 50% of the endothelial cells obtained by the method are VE-CADH positive, for example at least 60% of the endothelial cells obtained by the method are VE-CADH positive, such as at least 70% of the endothelial cells, for example such as 20 to 40% of the endothelial cells obtained by the method are VE-cadherin (VE-CADH, CD144) positive, or 20 to 70% of the endothelial cells obtained by the method are VE-CADH positive, such as 40 to 70% of the endothelial cells obtained by the method are VE-CADH positive, for example 50 to 70% of the endothelial cells obtained by the method are VE-CADH positive, such as 60 to 70% of the endothelial cells obtained by the method are VE-CADH positive.
Embodiment 31. The method according to any of the preceding embodiments, wherein at least 20% of the endothelial cells obtained by the method are cluster of differentiation 31 (CD31) positive, such as at least 30% of the endothelial cells obtained by the method are CD31 positive, for example at least 40% of the endothelial cells obtained by the method are CD31 positive, such as at least 50% of the endothelial cells obtained by the method are CD31 positive, for example at least 60% of the endothelial cells obtained by the method are CD31 positive, such as at least 70% of the endothelial cells, for example at least 80% of the endothelial cells obtained by the method are CD31 positive, such as 20 to 80% of the endothelial cells obtained by the method are CD31 positive.
Embodiment 32. The method according to any of the preceding embodiments, wherein about 0% of the endothelial cells obtained by the method are negative for Alpha-fetoprotein (AFP) markers.
Embodiment 33. The method according to any of the preceding embodiments comprising a further step of isolating CD144 positive and/or CD31 positive cells, such as by MACS or FACS sorting, such as by isolating CD144 positive cells.
Embodiment 34. The method according to any of the preceding embodiments, wherein the endothelial cells are positive for at least one marker selected from the list consisting of: VE-cadherin (VE-CADH, CD144) positive, and the combination of CD31 positive and VE-cadherin (VE-CADH, CD144) positive cells.
Embodiment 35. Endothelial cells obtained by the method according to any one of the preceding embodiments.
Embodiment 36. A composition comprising the endothelial cells obtained by the method according to any one of the preceding embodiments and cells of a further cell type.
Embodiment 37. The composition of embodiment 36, wherein said further cell type is hepatic progenitor cells, progenitor cells of endodermal, mesodermal or ectodermal origin, and a somatic differentiated cells type.
Embodiment 38. Kit of parts comprising:
   (i) a cell culture substrate comprising:laminin-521 or a fragment of laminin-521 and laminin-111 or a fragment of laminin-111; and
   (ii) at least one culture medium comprising one or more differentiation factors that stimulates endothelial cell differentiation of said pluripotent stem cells.
Embodiment 39. The kit according to embodiment 38, wherein the concentration of laminin-521 and laminin-111 in said cell culture substrate is in the range of about 1 to 10 microgram(s)/ml, for example about 3 to 8 microgram(s)/ml, such as 4 to 7 microgram(s)/ml, for example 5 to 6 microgram(s)/ml, such as about 5 ug/ml, such as 1.25 µg laminin-521/ml and 3.75 µg laminin-111/ml.
Embodiment 40. The kit according to any one of embodiments 38 or 39, wherein concentration of laminin-111 in said cell culture substrate is equal to or higher than the concentration of laminin-521, such as the ratio of laminin-521 to laminin-111 is in the range of 1:1 to 1:4, such as about 1:3.
Embodiment 41. The kit according to any one of embodiments 38 to 40, wherein said at least one culture medium comprises CHIR99021 (CHIR) and/or Bone morphogenetic protein 4 (BMP4).
Embodiment 42. The kit according to any one of embodiments 38 to 41, wherein said at least one culture medium comprises CHIR99021 (CHIR) within the range of 7 to 10 µM CHIR99021 (CHIR), such as 7 to 9 µM CHIR99021, for example 8 to 9 µM CHIR99021, such as about 8 µM CHIR99021.
Embodiment 43. The kit according to any one of embodiments 38 to 42, wherein said at least one culture medioum comprises Bone morphogenetic protein 4 (BMP4) at concentration within the range of 15 - 35 ng per ml medium, such as 25 - 30 ng Bone morphogenetic protein 4 (BMP4) per ml medium, such as about 25 ng Bone morphogenetic protein 4 (BMP4) per ml medium.
Embodiment 44. The kit according to any one of embodiments 38 to 43, wherein said at least one culture medium comprises Vascular endothelial growth factor (VEGF) and forskolin.
Embodiment 45. The kit according to any one of embodiments 38 to 44, wherein said at least one culture medium comprises Vascular endothelial growth factor (VEGF) at a concentration within a range of 100 to 300 ng/ml, such as 150 to 250 ng/ml, such as about 200 ng Vascular endothelial growth factor (VEGF)/ml.
Embodiment 46. The kit according to any one of embodiments 38 to 45, wherein said at least one culture medium comprises forskolin at a concentration within a range of 1 to 3 µM, such as about 2 µM.
Embodiment 47. The kit according to any one of embodiments 38 to 46, comprising
   a culture medium comprising CHIR99021 (CHIR) and Bone morphogenetic protein 4 (BMP4), and
   a further culture medium comprising Vascular endothelial growth factor (VEGF) and forskolin.
Embodiment 48. The kit according to embodiment 47, wherein said culture medium is a 1:1 mixture of DMEM:F12 and CTS Neurobasal medium comprising CHIR99021 (CHIR) and Bone morphogenetic protein 4 (BMP4), such as about 8 µM CHIR99021 and about 25 ng Bone morphogenetic protein 4 (BMP4).
Embodiment 49. The kit according to embodiment 47 or 48, wherein said further culture medium is StemPro-34^{™} comprising Vascular endothelial growth factor (VEGF) and forskolin, such as about 200 ng Vascular endothelial growth factor (VEGF)/ml and about 2 µM forskolin.
Embodiment 50. The kit according to any one of embodiments 47 to 49, wherein the concentration of laminin-521 and laminin-111 in said cell culture substrate is about 5 ug/ml, such as 1.25 µg laminin-521/ml and 3.75 µg laminin-111/ml.
Embodiment 51. The kit according to any one of embodiments 47 to 50, wherein ratio of laminin-521 to laminin-111 in said cell culture substrate is about 1:3.
Embodiment 52. The kit according to any one of embodiment 47 to 51, wherein said culture medium is a serum free medium.
Embodiment 53. Use of the cells embodiment 35 or the composition of embodiment 36 or 37 for the preparation of an aggretation of cells.
Embodiment 54. The use according to embodiment 53, wherein said aggregation is an aggregation of endothelial cells and hepatic progenitor cells.
Embodiment 55. A method for producing an aggregation of liver cells, said method comprising the step of:
   (i) providing endothelial cells obtained by the method according to any one of preceding embodiments;
   (i) providing hepatic progenitor cells;
   (ii) prepare a mixture comprising said hepatic progenitor cells and endothelial cells in a culture medium;
   (iii) seed the mixture on a cell culture substrate;
   (iv) allow the mixture of cells to propagate and form an aggregation of cells;
   (v) collect said aggregation of cells.
Embodiment 56. The method according to embodiment 55, wherein the ratio of hepatic progenitor cells to endothelial cells in the mixture is in the range of 1:1 to 4:1, such as 1:1 to 3.5:1, for example 1:1 to 3:1, for example 3.3:1. Embodiment 57. The method according to embodiment 55 or 56, wherein the endothelial cells are enriched for VE-cadherin (VE-CADH, CD144) positive cells.
Embodiment 58. The method according to any one of embodiments 55 to 57, wherein at least 20% of the endothelial cells are VE-cadherin (VE-CADH, CD144) positive, such as at least 25% of the endothelial cells are VE-cadherin (VE-CADH, CD144) positive, for example at least 30% of the endothelial cells are VE-cadherin (VE-CADH, CD144) positive, such as 20 to 40% of the endothelial cells VE-cadherin (VE-CADH, CD144) positive.
Embodiment 59. The method according to any one of embodiments 55 to 58, wherein 90-100% of the hepatic progenitor cells are positive for AFP and 90-100% of the hepatic progenitor cells are positive for HNF4a, such as 95-100% positive for AFP and 95-100% positive for HNF4a.
Embodiment 60. The method according to any one of embodiments 55 to 59, wherein the culture medium comprises 10 µM Y-27632 (ROCKi), 50 ng/ml VEGF (1:200), 10 ng/ml EGF (1:1000), 10 ng/ml FGF (1:1000), 10 ng/ml HGF (1:1000) and 20 ng/ml OSM (1: 1000).
Embodiment 61. The method according to any one of embodiments 55 to 60, wherein said cell culture substrate is a mould, such as an agarose mould.
Embodiment 62. The method according to any one of embodiments 55 to 61, wherein said mixture comprising said hepatic progenitor cells and endothelial cells is seeded on the center of said mould.
Embodiment 63. The method according to any one of embodiments 55 to 62, wherein said mixture of cells are allowed to propagate until the volume of cells exceeds the volume of the mould and subsequently transferring the cells to a well comprising culture medium, such as a mixture of William's and SFM endothelial media, for example a 1:1 mixture of William's and SFM endothelial media.
Embodiment 64. The method according to any one of embodiments 55 to 63, wherein said mixture comprising said hepatic progenitor cells and endothelial cells are allowed to propagate for about 5 to 8 days, such as about 7 days and subsequently transferring the cells to a well comprising culture medium for further propagation.
Embodiment 65. The method according to any one of embodiments 55 to 64, wherein said well is coated with 2-hydroxyethyl methacrylate (poly-HEMA).
Embodiment 66. The method according to any one of embodiments 55 to 65, wherein the liver cells are positive for HNF4a and display CYP1A2 function and albumin and A1AT secretion.
Embodiment 67. An aggregation of liver cells obtained by the method according to embodiments 55 to 66.
Embodiment 68. The aggregation of livers cells according to embodiment 67, wherein the liver cells are positive for HNF4a and display CYP1A2 function and albumin and A1AT secretion.
Embodiment 69. The aggregation of liver cells obtained by the method according to embodiments 55 to 65 for use as a medicament.
Embodiment 70. The aggregation of liver cells obtained by the method according to embodiments 55 to 65 for use in the treatment of acute-on-chronic liver failure (ACLF), acute liver failure, chronic liver failure, preparation for liver implantation, tyrosinemia type I or Alpha-1 antitrypsin deficiency (A1AD or AATD).
Embodiment 71. The method according to any one of embodiments 55 to 66, wherein said hepatic progenitor cells have been obtained by differentiating pluripotent stem cells, preferably human pluripotent stem cells, to hepatic progenitor cells by culturing the pluripotent stem cells on a cell culture substrate comprising laminin 521 and laminin 111 (or functional fragments of thereof) and contacting the cells with differentiation factors that induce the hepatic differentiation.
Embodiment 72. The method according to any one of embodiments 55 to 66 or 71, wherein said hepatic progenitor cells have been hepatic progenitor cells have been obtained by seeding pluripotent stem cells on a cell culture substrate comprising a mixture of laminin-521 or a fragment thereof and laminin-111 or a fragment thereof; and culturing the pluripotent stem cells to obtain said hepatic progenitor cells.
Embodiment 73. The method according to any one of embodiments 55 to 66, 71-72, wherein said hepatic progenitor cells have been obtained by culturing the pluripotent stem cells in the presence of activin A and Wnt3a.
Embodiment 74. The method according to any one of embodiments 55 to 66, 71-73, wherein said hepatic progenitor cells have been obtained by culturing the pluripotent stem cells in the presence of activin A and Wnt3a followed by culturing the pluripotent stem cells in the presence of hydrocortisone (HC), hepatocyte growth factor (HGF), and oncostatin m (OSM).

### Examples

### Example 1 - Preparation of endothelial cells

Pluripotent stem cells (PSCs) were expanded on laminin 521 or a blend of laminin extracellular matrices (521 and 111). Following this pluripotent stem cells were removed from their matrix using cell dissociation reagent and replated on to laminin extracellular matrix containing laminins 521 and 111. When the PSCs reached the correct density, they were exposed to mesodermal priming medium containing CHIR and BMP4 for 72 hours. Following this, cells were incubated in endothelial differentiation medium containing VEGF and forskolin for 48 hours (with a medium change at 24 hours). Representative cell morphologies are shown in Figure 1 and endothelial cell marker immunostaining shown in Figures 2 and 3. Endothelial cell populations were subsequently removed from their matrix and enriched using CD144 magnetic beads and then used for liver tissue engineering purposes.

The protocol is outlined in further details below:
***Day* -1** (corresponding to day 3 of hepatic progenitor cell differentiation)
   1. Aspirate the medium from hESCs at 60-80% confluency.
   2. Wash the cells with 1 mL DPBS (without CaCl₂⁺/MgCl₂⁺, *Thermo Fisher*) at room temperature (RT) per 10 cm² (for example, for a T175 flask, wash with 17.5 mL of PBS).
   3. Aspirate the DPBS from the flask.
   4. Add 1ml of Gentle Cell Dissociation Reagent (GCDR, *STEMCELL Technologies*) per 10 cm² and leave the cells at in 37°C/5% COz cell culture incubator for 7 minutes to dissociate into single cells.
   5. After 7 minutes, stop the reaction by adding supplemented mTeSR^{™} Plus with 10 µM Rho-associated kinase (ROCK) inhibitor Y-27632 (here referred as Rocki). The volume added must be the same as the GCDR volume added in the previous step.
   6. To achieve a single cell suspension, pipette gently up and down 10-20 times.
   7. Centrifuge the suspension at 300 G for 5 minutes at RT.
   8. Aspirate the supernatant carefully without disturbing the cell pellet.
   9. Add 2 mL of pre-warmed supplemented mTeSR^{™} Plus with 10 µM ROCKi to the cells and gently pipette up and down to ensure a homogeneous single cell suspension.
   10.Take a 50 µL sample from the single cell suspension and dilute it in 200 µL of DPBS in an Eppendorf tube. Count the total number of live cells using an automatic cell counter.
   11.Prepare a falcon tube with mTeSR^{™} Plus supplemented with 10 µM ROCKi. The volume needed is 3 times the surface of the flask seeded (for example: for a T175 flask, the volume needed is 52.5 mL)
   12.From the tube with the single cell suspension (step 9), take the corresponding volume to seed the cells in a density of 30,000 cells per cm², and add it to the tube with mTeSR1^{™} Plus (step 11). For example: to seed a T175, the number of total cells needed is 5.25×10⁶ (5.25M cells).
   13.Seed the cells into a pre-warmed LN521/LN111 (1:3) coated flask.
   14.Incubate the plate in a 37°C/5% COz cell culture incubator for 24 h to start the differentiation to endothelial cells.
***Day 0***
   15. After 24 h, cells should have reached a confluency of around 40% and show a net-like shape.
   16.Replace the medium with 343 µL/cm² of mesoderm priming medium supplemented with CHIR99021 (1:1200) and BMP4 (1:2000) per cm². For example: for a T175 flask, the volume of supplemented mesoderm priming medium needed is 60 mL. Incubate at 37°C/5% COz for 72 h.
***Day 3***
   17. After 72 h, replace the medium with 215 µL/cm² of StemPro-34 media supplemented with VEGF (1:50) and forskolin (1:5000). Incubate at 37°C/5% CO₂ for 24 h.
***Day 4***
   18. After 24 h, replace the medium as described in step 17.
***Day 5***
   Collect the cells for sphere aggregation - explained in the protocol called "sphere aggregation".

### Example 2 - Endothelial cell differentiation efficiency

Endothelial cell differentiation efficiency. Table 1 below shows the number of total cells obtained after 5 days of endothelial cell differentiation in five independent experiments. The average total number of cells obtained is around 400.000 cells per cm², of which between ~21% to 37% are CD144 positive.

**Table 1**

| Differentiation experiment | Initial flasks | Total cells obtained | Isolated CD144+ cells | % of cells CD144+ | Total cells/cm² |
|---|---|---|---|---|---|
| 1st | 1x T175 | 70 x 10⁶ | 24,3 x 10⁶ | 34,7% | 400.000 |
| 2nd | 2x T175 | 136,4 x 10⁶ | 32,3 x 10⁶ | 23,6% | 389.700 |
| 3rd | 2x T175 | 136,5 x 10⁶ | 51,08 x 10⁶ | 37,4% | 390.000 |
| 4th | 1x T75 1x T25 | 48,69 x 10⁶ | 15,52 x 10⁶ | 31,87% | 486.900 |
| 5th | 2x T175 | 30 x 10⁶ | 30 x 10⁶ | 21% | 403.200 |

### Example 3 - Liver sphere aggregation and maintenance

### SPHERE AGGREGATION PROTOCOL

Before starting, prepare 0.5% BSA/PBS and filter by using a 0.22 µm filter. Once filtered, place it in the fridge (4°C). Solution must be cold before using it for magnetic separation. This solution must be prepared on the same day as sphere aggregation.

### Endothelial cell preparation

1. Endothelial cells are produced in accordance with the method disclosed herein.
2. To collect the cells for CD144 enrichment using MACS (*Miltenyi*), wash the cells with 1 mL of DPBS per 10 cm² (*for example*: for a T175 flask, wash with 17.5 ml of PBS) at RT.
3. Aspirate the DPBS from the flask.
4. Add 1 mL of pre-warmed TrypLE Express Enzyme (here referred as TRYPLE) per 10 cm² and leave the cells in a 37°C/5% COz cell culture incubator for 7 minutes to dissociate into single cells.
5. After 7 minutes, stop the reaction by adding liver sphere medium with 10 µM ROCKi. The volume added must be the same as the TRYPLE volume added in the previous step.
6. To achieve a single cell suspension, pipette gently up and down 10-20 times.
7. Filter the cell suspension through a 100 µm filter.
8. Filter the cell suspension through a 70 µm filter.
9. Filter the cell suspension through a 30 µm filter.
10. Count the cells using an automatic cell counter. For more accuracy, make three independent counts.
11. Spin down cell suspension by centrifuging the cells at 300 G for 10 minutes.
12. MACS enrichment is performed as recommended by manufacturer's instructions. Briefly, resuspend a cell pellet in 80 µL of PBS 0.5% BSA per 107 cells and add 20 µL of CD144-magnetic beads. Mix well and incubate at 4°C for 15 min. Wash cells with 1.5 mL of cold DPBS with 0.5% BSA and centrifuge the cell suspension at 300 G for 10 minutes.
13. Resuspend up to 10⁸ cells in 500 µL of DPBS with 0.5% BSA.
14. Prime the column by washing the LS column with 3 mL of DPBS with 0.5% BSA.
15. Apply the cell suspension onto the column. Flow through contains the unlabelled cells.
16. Wash the column three times with 3 mL of DPBS with 0.5% BSA.
17. Remove the LS column from the magnetic separator and add 5 mL of DPBS with 0.5% BSA. Quickly collect the magnetically labelled cells by pushing the plunger into the column.
18. Count cells using an automatic cell counter to calculate the yield of CD144 positive endothelial cells. For more accuracy, make three independent counts.
19. Spin down cell suspension at 300 G for 10 minutes.
20. Resuspend the cell pellet at 4.608 × 10⁶ cells/mL in the liver sphere medium supplemented with 10 µM ROCKi, VEGF (1:200), EGF (1:1000), FGF (1:1000), HGF (1:1000) and OSM (1:1000).
21. If endothelial cells are not used immediately, keep in the fridge (4°C) for a maximum of 2h.

### Hepatic progenitor cell preparation

1. Provide hepatic progenitor cells, such as hepatic progenitor produced in line with WO2017072580A1.
2. To collect the cells for sphere aggregation, wash the cells with 1 mL of DPBS per 10 cm² (*for example:* for a T175 flask, wash with 17.5 mL of PBS) at RT.
3. Aspirate the DPBS from the flask.
4. Add 1 mL of pre-warmed TRYPLE per 10 cm² and leave the cells at in 37°C/5% CO2 cell culture incubator for 30-35 minutes to dissociate into single cells.
5. After 30-35 minutes, stop the reaction by adding liver sphere medium with 10 µM ROCKi. The volume added must be the same as the TRYPLE volume added in the previous step.
6. To achieve a single cell suspension, pipette gently up and down 10-20 times.
7. Filter the cell suspension through a 100 µm filter.
8. Filter the cell suspension through a 70 µm filter.
9. Filter the cell suspension through a 30 µm filter.
10. Count the cells using an automatic cell counter. For more accuracy, make three independent counts.
11. Spin down cell suspension by centrifuging the cells at 300 G for 10 minutes.
12. Resuspend the cell pellet at 7.68 × 10⁶ cells/mL in liver sphere medium supplemented with 10 µM ROCKi, VEGF (1:200), EGF (1:1000), FGF (1:1000), HGF (1:1000) and OSM (1:1000).

### Micromoulds (3D green microtissues) preparation

1. In the hood, place a plastic cover (it can be from the 6-well plate) upside-down. It will be needed to place the green 3D microtissues on top of that so they're not moving and do not touch the bottom of the hood.
2. Prepare 2% agarose powder in MilliQ/sterile water (i.e 2 g in 100 mL water).
3. Dissolve in the microwave and let it cool down.
4. Add 520 µL of the dissolved agarose directly on the top of the 3D microtissues (under the hood).
5. Let them cool down 10-15 min at RT. Make sure they're quite solid so they do not break when taken out from the green mould.
6. In the meantime, prepare 12-well plate with 1 mL PBS CaCl₂⁺MgCl₂⁺/well (under the hood).
7. Once the micromoulds are ready, turn them gently upside-down and place each of them directly to the well filled with 1 mL PBS CaCl₂⁺MgCl₂⁺. Once done, cover the transparent moulds with the additional 0.5 mL PBS CaCl₂⁺MgCl₂⁺.
8. Seal the 12-well plate containing micromoulds with the parafilm. Keep in the fridge maximum 2 months.

### Cell mix suspension and dispensation to 3D moulds

For every agarose mould with 256 microwells (Sigma-Aldrich), 100 µL of hepatic progenitor cell suspension at 7.68 × 10⁶ cells/mL, 50 µL of our CD144 enriched endothelial cell suspension at 4.608 × 10⁶ cells/mL and 40 µL of liver sphere medium with 10 µM of ROCKi must be added to a falcon tube.
1. Prepare the cell mix suspension that you have calculated for the number of moulds you have and ensure you have 5% extra volume.
2. Ensure the agarose moulds with PBS have been at least 20 minutes in the incubator at 37°C.
3. Aspirate as much PBS from inside and outside of the moulds of each well of a 12 well plate.
4. Dispense 190 µL of the cell mix suspension to the center of agarose mould and left in the incubator at 37°C for 2 hours for the cells to decant.
5. After 2 hours, add 1.5 mL of liver sphere medium supplemented with 10 µM ROCKi to the side of each well of a 12-well plate, ensuring that it is not added directly onto microwells of the mould.
6. After 24 hours, remove all the media from the outside of the mould (inside each well of the 12-well plate) and add 1.5 mL of liver sphere medium, change 1 mL of the medium every other day.

1. After approximately 7 days, spheres will become too large and therefore automatically pop out of the mould. Spheres can be removed from the mould by pipetting liver sphere medium directly over the microwells.
2. Add liver sphere medium directly over the microwells of the mould and ensure, with the help of an optic microscope, that all spheres have been removed from wells.
3. Transfer by pipetting either all (256) spheres to a poly-HEMA coated 12-well plate or approximately a third (equivalent to 85) of the total number of spheres from 1 mould to a poly-HEMA coated 48-well plate.
4. Add approximately 500 µL per 85 spheres in a poly-HEMA coated 48-well plate. From there, every second day remove 250 µL of used media and add 250 µL of fresh liver sphere medium.

A 100 µm pluristrainer mini (*Pluriselect*) is used (weeks 0-3) as a filter for the tip to perform the media change to ensure that none of the spheres is unintentionally aspirated. From week 3 onwards, a 200 µm pluristrainer mini (*Pluriselect*) can be used to safely aspirate medium.

### Coating poly-HEMA plates

Spheres must be transferred to poly-HEMA coated wells after one week in the green micromoulds (3D micromoulds, Sigma-Aldrich).
1. Dissolve 1 g of poly 2-hydroxyethyl methacrylate (poly-HEMA) in 50 mL of 97% ethanol.
2. Stir the solution overnight using a hot plate at 60°C.
3. Add 250 µL of the solution per well of a 24-well plate.
4. Let dry for 24-48h at 50°C. Coated plates can be stored up to 3 months at room temperature. Seal with parafilm to avoid contamination.

### Sample (supernatant) retrieval from spheres for ELISA analysis

For these steps, the end of the p1000 tip must be cut to increase the aperture diameter. This prevents spheres from clogging the end of the tip and damaging cells. *Important:* To perform these assays, only William's medium is added in order not to interfere with the ELISA and CYPs assay's background signal.
1. Transfer all spheres with used liver sphere medium to an Eppendorf tube.
2. Wait 3-5 minutes for the spheres to decant in the bottom.
3. Manually aspirate all medium without disturbing the decanted spheres.
4. Add 500 µL of William's medium (without growth factors and supplements).
5. Wait 3-5 minutes for the spheres to decant in the bottom.
6. Gently aspirate all the William's medium without disturbing the decanted spheres.
7. Add William's medium supplemented with growth factors and substrates at the respective concentrations indicated by the manufacturers.
8. Transfer spheres with medium back to their original poly-HEMA coated well.
9. After 24 hours transfer all spheres with used liver sphere medium to an Eppendorf tube.
10. Wait 3-5 minutes, or until the spheres decant in the bottom.
11. Gently transfer all the used medium without disturbing the decanted spheres to a labelled Eppendorf tube and freeze at -20°C.
12. Add liver sphere medium (1: 1 SFM and William's medium).
13. Transfer spheres with fresh medium back to their original poly - HEMA coated well.

### Results

Pluripotent stem cells (PSCs) were expanded on laminin 521 or a blend of laminin extracellular matrices (521 and 111). When the PSCs reached the correct density, they were exposed to endodermal priming medium containing Activin A and Wnt3a for 72 hours and then hepatic progenitor specification medium containing 1% DMSO for 5 days, with medium changes every 48 hours. Stem cell progenitors are removed from their matrix and subsequently used for tissue engineering.

To prime toward the mesoderm, PSCs were exposed to mesodermal priming medium containing CHIR99021 and BMP4 for 72 hours. Following this, cells were incubated in endothelial differentiation medium containing VEGF and forskolin for 48 hours (with a medium change at 24 hours). Endothelial cell populations were subsequently removed from their matrix and enriched using CD144 magnetic beads and then used for liver tissue engineering purposes.

Liver tissue engineering is achieved by cell self-assembly, using microwell technology. PSC derived hepatic progenitors and endothelial cells were removed from their ECMs (laminin 521 vs laminin 521 and 111) and then mixed at the ratio 3: 1 and form liver spheres over time (Figure 4).

Stem cell derived liver tissue is functional over at least a 6-week period. During this time, the inventors observed maintenance of mature liver functions (albumin secretion and cytochrome P450 activity) when fetal liver protein secretion (AFP) decreased (Figures 5 to 7). Notably, stem cell derived liver tissue produced using the blend of laminins 521 and 111 is most mature by weeks 5 and 6, displaying significantly reduced AFP secretion (approaching baseline) and significantly increased levels of adult enzyme activity, cytochrome P450 1A2.

**Table 2. List of growth factors used for liver sphere aggregation and maintenance.**

| **Name** | **Stock** | **Dilution** | **Working concentration** | **Supplier** |
|---|---|---|---|---|
| **Rocki (Y-27632)** | 10 mM | 1:1000 | 10 µM | Bio-Techne |
| **VEGF (liver spheres)** | 50 µg/mL | 1:200 | 50 ng/mL | Bio-Techne |
| **Oncostatin M (OSM)** | 20 µg/mL | 1:1000 | 20 ng/mL | PeproTech |
| **Human EGF** | 10 µg/mL | 1:1000 | 10 ng/mL | PeproTech |
| **Human HGF** | 10 µg/mL | 1:1000 | 10 ng/mL | PeproTech |
| **Human FGF basic** | 10 µg/mL | 1:1000 | 10 ng/mL | PeproTech |

**Table 3. Major media, supplements, and additional items used for liver sphere aggregation and maintenance.**

| **Liver spheres** | | | |
|---|---|---|---|
| Liver sphere medium | William's E medium | 500 mL | Thermo Fisher |
| | SFM-Endothelial medium | 500 mL | Thermo Fisher |
| | KOSR Serum Replacement | 5% | Life Technologies |
| | Glutamax | 1% | Life Technologies |
| | Penicillin-streptomycin (10.000 U/mL) | 1% | Life Technologies |
| William's medium (for assays) | William's E medium | 500 mL | Thermo Fisher |
| | KOSR Serum Replacement | 10% | Life Technologies |
| | Glutamax | 1% | Life Technologies |
| | Penicillin-streptomycin (10.000 U/mL) | 1% | Life Technologies |
| Additional items | 2-hydroxyethyl methacrylate | | Sigma-Aldrich |

Stem cell derived liver spheres produced using the blend of laminins 521 and 111 displayed moderate and consistent growth over the days as shown in *Figure 4**.* These liver spheres did not have any visible necrotic cores, as opposed to the liver spheres derived from cells grown in only LN521. Furthermore, spheres grown in LN521 displayed a greater number of smaller spheres and were smaller on average compared to the other conditions. Liver spheres produced using the mix showed no visible difference with the spheres grown in the blend. (see *Figure 4*)*.*

AFP secretion of free-floating liver spheres was measured over the course of 6 weeks for the blend, LN521 and mix conditions, as indicated in *Figure 5**.* From week 2 onwards, blend-produced liver spheres consistently decreased AFP secretion down to baseline values. However, LN521-produced spheres, on average, followed a trend, initiating with a relatively low secretion of AFP at week 1 maintaining its secretion over weeks 2 and 3, decreasing it at week 4 and then increasing at week 5 and finally reducing by week 6. Lastly, spheres produced using the mix followed an increasing trend peaking at week 3, then decreasing and maintaining AFP secretion to a relatively high amount at weeks 4 and 5 and finally decreasing it on week 6. Since AFP is a marker of early hepatic maturity, the inventors found that its consistent and decreasing secretion in the blend condition. The LN521 and mix conditions, even if resulting in low secretions in week 6, showed less consistent decreases over previous weeks. This is undesirable as it could potentially lead to a less mature and consistent cellular product *(**Figure 5**).*

Albumin (ALB) secretion of free-floating liver spheres was measured over the course of 6 weeks for the blend, LN521 and mix conditions, as indicated in *Figure 6**.* From week 2 onwards, blend-produced liver spheres consistently increased their ALB secretion until week 6. LN521-produced liver spheres secreted detectable amounts of ALB from week 2 onwards as well. However, spheres derived from LN521 displayed less consistent albumin secretion with decreases observed at weeks 3 and week 4. The spheres produced from LN521 in weeks 5 and 6 increased albumin secretion. Lastly mix-produced spheres seem to have a bimodal trend of albumin secretion. At week 1 a significant amount of ALB was secreted when compared to the other conditions at this week. Then, on average, the trend of ALB secretion decreases at week 2, only to increase again until week 5. At week 6, liver spheres produced from mix displayed decreased secretion. ALB is a serum protein produced by mature hepatocytes. The blend condition, on average, showed a consistent and increasing levels of this marker. The LN521 and mix conditions showed less consistent increases over previous weeks. This is undesirable as it could potentially lead to a less reliable cellular product *(**Figure 6**).*

CYP1A2 is a neonatal/adult cytochrome P450 enzyme that catalyzes many reactions involved in drug metabolism, synthesis of cholesterol and other lipids. The liver sphere's activity of this enzyme was measured over 5 weeks for the conditions of blend, LN521, and mix as shown in *Figure 7**.* The enzymatic activity was measured for 5 weeks for all 3 conditions. During the first 3 weeks, the activity consistently increased, showing no significant differences between any of the conditions in the same week. At week 5, the mix-derived liver spheres showed the highest activity, followed by blendderived spheres, and lastly LN521 derived spheres *(**Figure 7**).*

## Claims

1. A method for producing endothelial cells, comprising:
seeding pluripotent stem cells on a cell culture substrate comprising:
(i) laminin-521 or a fragment of laminin-521;
(ii) laminin-111 or a fragment of laminin-111; and
culturing the pluripotent stem cells, such as human pluripotent stem cells, in the presence of one or more differentiation factors that stimulates endothelial cell differentiation of said pluripotent stem cells to obtain said endothelial cells.

2. The method according to claim 1, wherein the concentration by weight of laminin-111 in said cell culture substrate is equal to or higher than the concentration of laminin-521, such as the ratio of laminin-521 to laminin-111 is in the range of 1:1 to 1:4, such as about 1:3.

3. The method according to claim 1 or 2, wherein the concentration of laminin-521 and laminin-111 in said cell culture substrate is in the range of about 1 to 10 microgram(s)/ml, for example 3 to 8 microgram(s)/ml, such as 4 to 7 microgram(s)/ml, for example 5 to 6 microgram(s)/ml, such as about 5 ug/ml, such as 1.25 µg laminin-521/ml and 3.75 µg laminin-111/ml.

4. The method according to any of the preceding claims, wherein CHIR99021 (CHIR) and/or Bone morphogenetic protein 4 (BMP4) is added to the cells about 12 to 48 h after seeding the pluripotent stem cells on a cell culture substrate, such as 24 to 48 h after seeding the pluripotent stem cells on a cell culture substrate, for example about 24 h after seeding the pluripotent stem cells on a cell culture substrate.

5. The method according to any of the preceding claims, wherein Vascular endothelial growth factor (VEGF) and/or forskolin is added to the cells about 48 to 96 h after seeding the pluripotent stem cells, such as 48 to 72 h after seeding the pluripotent stem cells, for example about 72 h after seeding the pluripotent stem cells.

6. The method according to any one of the preceding claims , wherein culture medium comprising Vascular endothelial growth factor (VEGF) and/or forskolin replaces the culture medium comprising CHIR99021 (CHIR) and/or Bone morphogenetic protein 4 (BMP4).

7. The method according to any of the preceding claims, wherein the pluripotent stem cells are seeded on said culture substrate at a density of 25000 to 40000 pluripotent stem cells per cm², such as 30000 to 35000 pluripotent stem cells per cm², for example 30000 pluripotent stem cells per cm².

8. The method according to any of the preceding claims, said method comprising:
seeding pluripotent stem cells on a cell culture substrate comprising: (i) laminin-521 or a fragment of laminin-521;
(ii) laminin-111 or a fragment of laminin-111; and
culturing the pluripotent stem cells in a first culture medium for about 24 hours or until the cells are about 40% confluent;
replacing the first culture medium with a second culture medium comprising CHIR99021 (CHIR) and Bone morphogenetic protein 4 (BMP4) and culture the cells for about 72 hours;
replacing the second culture medium comprising with a third culture medium comprsing VEGF and forskolin and culture the cells for about 48 hours.

9. The method according to any of the preceding claims comprising a further step of isolating VE-cadherin (VE-CADH, CD144) positive and/or CD31 positive cells, such as by MACS or FACS sorting, such as by isolating VE-cadherin (VE-CADH, CD144) positive cells.

10. Endothelial cells obtained by the method according to any one of the preceding claims.

11. Kit of parts comprising:
(i) a cell culture substrate comprising:laminin-521 or a fragment of laminin-521 and laminin-111 or a fragment of laminin-111, optional wherein concentration of laminin-111 in said cell culture substrate is equal to or higher than the concentration of laminin-521, such as the ratio of laminin-521 to laminin-111 is in the range of 1:1 to 1:4, such as about 1:3; and
(ii) at least one culture medium comprising one or more differentiation factors that stimulates endothelial cell differentiation of said pluripotent stem cells, such as a culture medium comprising CHIR99021 (CHIR) and Bone morphogenetic protein 4 (BMP4), and a further culture medium comprising Vascular endothelial growth factor (VEGF) and forskolin.

12. A method for producing an aggregation of liver cells, said method comprising the step of:
(i) providing endothelial cells obtained by the method according to any one of preceding claims;
(i) providing hepatic progenitor cells;
(ii) prepare a mixture comprising said hepatic progenitor cells and endothelial cells in a culture medium;
(iii) seed the mixture on a cell culture substrate, such as a mould;
(iv) allow the mixture of cells to propagate and form an aggregation of cells;
(v) collect said aggregation of cells.

13. The method according to claim 12, wherein the ratio of hepatic progenitor cells to endothelial cells in the mixture is in the range of 1:1 to 4:1, such as 1:1 to 3.5:1, for example 3:1 or 3.3:1.

14. An aggregation of liver cells obtained by the method according to claim 12 or 13.

15. The aggregation of liver cells obtained by the method according to claim 55 to 65 for use as a medicament, such as a medicament for use in the treatment of of acute-on-chronic liver failure (ACLF), acute liver failure, chronic liver failure, preparation for liver implantation, tyrosinemia type I or Alpha-1 antitrypsin deficiency (A1AD or AATD).
